# EUROPÄISCHE PATENTANMELDUNG

(11) **EP 4 840 447 A1**
(43) Veröffentlichungstag der Anmeldung: **18.11.2026**
(21) Anmeldenummer: 25176438.7
(22) Anmeldetag: 14.05.2025
(51) Int. Cl.: A61K 31/198, A61K 31/519, A61P 15/10, A61P 17/14

(54) **ZUSAMMENSETZUNG UMFASSEND SILDENAFIL UND DIMETHYLGLYCIN**

(71) Anmelder: Dr. Kurt Wolff GmbH & Co. KG, 33611 Bielefeld (DE); Dr. August Wolff GmbH & Co. KG, 33611 Bielefeld (DE)
(72) Erfinder: SCHULZE ZUR WIESCHE, Erik, 33611 Bielefeld (DE); LAUTERBACH, Andreas, 33611 Bielefeld (DE); DOERRENBERG, Eduard R., 33611 Bielefeld (DE)
(74) Vertreter: Weickmann & Weickmann PartmbB

(57) **Zusammenfassung**

Die vorliegende Erfindung betrifft eine Zusammensetzung umfassend Sildenafil und/oder ein Salz davon und Dimethylglycin und/oder ein Salz davon, sowie eine derartige Zusammensetzung zur Verwendung bei der Vorbeugung und/oder Behandlung von Haarausfall und/oder sexuellen Dysfunktionen. Ferner betrifft die Erfindung ein Kit umfassend Sildenafil und/oder ein Salz davon und Dimethylglycin und/oder ein Salz davon, wobei die beiden Verbindungen in getrennten Verabreichungsformen vorliegen.

## Beschreibung

Die vorliegende Erfindung betrifft eine Zusammensetzung umfassend Sildenafil und/oder ein Salz davon und Dimethylglycin und/oder ein Salz davon, sowie eine derartige Zusammensetzung zur Verwendung bei der Vorbeugung und/oder Behandlung von Haarausfall und/oder sexuellen Dysfunktionen. Ferner betrifft die Erfindung ein Kit umfassend Sildenafil und/oder ein Salz davon und Dimethylglycin und/oder ein Salz davon, wobei die beiden Verbindungen in getrennten Verabreichungsformen vorliegen.

Eine ausreichende Durchblutung des gesamten Organismus ist essentiell zur Aufrechterhaltung des Zellstoffwechsels und der Gesundheit. Dementsprechend hängen viele Störungen und Erkrankungen des menschlichen Körpers mit einer unzureichenden lokalen Durchblutung eines Körperteils zusammen. Umgekehrt kann eine systemische oder lokale Förderung der Durchblutung solchen Störungen entgegenwirken.

Haarausfall kann die Lebensqualität der Betroffenen erheblich beeinflussen. Die Ursachen von Haarausfall sind vielfältig und reichen von altersbedingtem Haarausfall über genetisch bzw. hormonell bedingte Störungen, Autoimmunstörungen, Stoffwechselstörungen, einschließlich Fehlernährung, bis hin zu Infektionen, Traumen, Tumoren und Medikamenten wie Zytostatika und Antikoagulantien. Haarausfall umfasst verschiedene Erkrankungen, darunter androgenetische Alopezie, Telogenes Effluvium, Alopecia areata, frontale fibrosierende Alopezie und Lichen planopilaris.

Ein durch Hormone wie Androgene hervorgerufener Haarausfall wird als androgenetische Alopezie (Alopecia androgenetica oder auch AGA) bezeichnet und gilt insbesondere bei Männern, aber auch bei Frauen als die häufigste Ursache für Haarausfall. Hierbei kann zwischen männlichen und weiblichen Haarausfallmustern unterschieden werden. Andere häufige Formen von nicht vernarbender Alopezie umfassen Telogenes Effluvium (TE), einen diffusen Haarausfall, sowie den akut einsetzenden entzündlich bedingten kreisrunden Haarausfall, der auch als Alopecia areata (AA) bezeichnet wird.

Lichen planopilaris ist eine entzündliche Autoimmunerkrankung der Haut, die zu einer Zerstörung von Haarfollikeln und vernarbender Alopezie führt. Frontale fibrosierende Alopezie ist eine klinische Variante von Lichen planopilaris, die hauptsächlich bei Frauen nach den Wechseljahren auftritt.

Eine Stimulierung der Durchblutung der Kopfhaut fördert die lokale Mikrozirkulation und kann die Nährstoff- und Sauerstoffversorgung von Haarfollikeln verbessern. Verschiedene Studien haben gezeigt, dass eine erhöhte Durchblutung der Kopfhaut Haarwachstum fördern kann und Haarausfall vorbeugen und/oder wirksam behandeln kann (Choi et al., Biochem Biophys Res. Commun. (2018) 505, 685-691).

Eine weitere Gruppe von Störungen oder Erkrankungen, in denen lokale Durchblutung eine wichtige Rolle spielt, sind sexuelle Dysfunktionen, insbesondere sexuelle Erregungsstörungen. Männliche sexuelle Erregungsstörungen umfassen dabei insbesondere erektile Dysfunktionen. Weibliche sexuelle Erregungsstörungen umfassen genitale, subjektive und kombinierte Erregungsstörungen, wobei insbesondere genitale und kombinierte Erregungsstörungen mit einer unzureichenden Erhöhung der Durchblutung der Geschlechtsorgane zusammenhängen.

Sowohl männliche als auch weibliche sexuelle Dysfunktionen und insbesondere Erregungsstörungen können erfolgreich mit durchblutungsfördernden Wirkstoffen vorbeugend oder therapeutisch behandelt werden. Ein häufig eingesetzter Wirkstoff hierfür ist Sildenafil, auch bekannt unter dem Handelsnamen Viagra^{®}.

Sildenafil ist zugelassen für die Behandlung von erektiler Dysfunktion und pulmonal-arterieller Hypertonie. Der Wirkstoff wird häufig als Salz formuliert, beispielsweise als Sildenafilcitrat oder Sildenafillaktat.

Mechanistisch wirkt Sildenafil auf die NO-abhängige Vasodilatation durch Hemmung der cGMP-spezifischen Phosphodiesterase vom Typ 5 (PDE-5).

Vasodilatation bezeichnet die Ausdehnung oder Erweiterung der Blutgefäße, mit anderen Worten die Vergrößerung ihres Lumens. Die Vasodilatation ist eine physiologische Reaktion, die dazu führt, dass der Gefäßquerschnitt und damit die Durchblutung hinter dem betroffenen Gefäßabschnitt vergrößert wird. Auslösend ist eine Relaxation (Entspannung) der glatten Gefäßmuskulatur.

Stickstoffmonoxid (NO) wird durch die Umwandlung von L-Arginin in NO über NO-Synthasen (NOS) in verschiedenen Teilen des Körpers produziert. Die endotheliale NOS (eNOS) produziert NO, das explizit als endogener Vasodilatator wirkt, in die Gefäßzellen diffundiert und die Entspannung der glatten Muskulatur durch Bindung an die Guanylylzyklase vermittelt. Die Guanylylzyklase wandelt GTP in cGMP um, das die cGMP-abhängigen Proteinkinasen aktiviert. Diese Proteinkinasen phosphorylieren verschiedene Ionenkanäle im endoplasmatischen Retikulum (ER), wodurch Kalzium sequestriert und eine Kalziummobilisierung innerhalb der Zelle verhindert wird. Infolgedessen entspannen sich die glatten Muskelzellen (Jennifer Witek; Anand D. Lakhkar., Treasure Island (FL): StatPearls Publishing; 2024 Jan). Die Wirkung von NO wird durch die Aktivität der cGMP-spezifischen Typ 5-Phosphodiesterase (PDE-5) verringert, welche cGMP durch Umwandlung in GMP abbaut. Als spezifischer PDE5-Hemmer fördert Sildenafil cGMP-Signalgebung und Vasodilatation. Die vorliegende Erfindung ist nicht auf diesen Mechanismus limitiert.

Allerdings weist Sildenafil insbesondere bei hohen Dosierungen ein breites Nebenwirkungsprofil auf. Nebenwirkungen umfassen unter anderem Anämie, Wassereinlagerungen, Schlaflosigkeit, Unruhe, Kopfschmerzen, Migräne, Zittern, Parästhesie, Brennen, Hypästhesie, Netzhautblutungen, Sehstörungen, Schwindel, Gesichtsrötung, Nasenbluten, Durchfall und Dyspepsie.

Daher besteht weiterhin Bedarf an verbesserten Zusammensetzungen, die eine verbesserte Wirksamkeit und/oder Verträglichkeit zeigen und/oder die Durchblutung auch bei solchen Patienten fördern, die auf eine Behandlung mit Sildenafil allein nicht oder nicht ausreichend ansprechen. Insbesondere sind Zusammensetzungen wünschenswert, die ein verbessertes Nebenwirkungsprofil zeigen, um die regelmäßige Anwendung über einen längeren Zeitraum, z.B. zur Vorbeugung und/oder Behandlung von Haarausfall, zu erleichtern.

Diese Aufgabe wird im Rahmen der vorliegenden Erfindung überraschend durch die Kombination von Sildenafil mit Dimethylglycin (DMG) gelöst.

Ein erster Aspekt der Erfindung betrifft eine Zusammensetzung umfassend
(A) Sildenafil und/oder ein Salz davon, und
(B) Dimethylglycin und/oder ein Salz davon.

Bevorzugt ist die Zusammensetzung eine pharmazeutische Zusammensetzung.

Vorzugsweise ist die Komponente (A) ein Salz von Sildenafil, bevorzugt ein pharmazeutisch annehmbares Salz von Sildenafil. Pharmazeutisch annehmbare Salze von Sildenafil sind beispielsweise Sildenafilcitrat oder Sildenafillaktat. Sildenafilcitrat ist als Komponente (A) besonders bevorzugt.

Bevorzugt enthält die erfindungsgemäße Zusammensetzung Sildenafil und/oder ein Salz davon (A), insbesondere Sildenafilcitrat, in einer Menge von 0,001 bis 20,0 Gewichtsprozent (Gew.-%), bevorzugt 0,001 bis 15,0 Gew.-%, stärker bevorzugt 0,01 bis 10,0 Gew.-%, noch stärker bevorzugt 0,1 bis 5,0 Gew.-% und besonders bevorzugt 0,1 bis 1,5 Gew.-%.

Falls nicht anders angegeben, beziehen sich sämtliche Angaben in Gew.-% in der vorliegenden Offenbarung jeweils auf das Gesamtgewicht der Zusammensetzung.

Wie hierin verwendet, ist der Begriff "Dimethylglycin" synonym mit "*N,N-*Dimethylglycin", "Dimethylaminoessigsäure" oder der Abkürzung "DMG". Dimethylglycin entspricht der folgenden Formel (I):

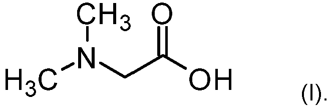

DMG kommt in Pflanzen, Tieren und dem Menschen vor, wobei es im Menschen nur in sehr geringen Mengen gebildet wird. Es entsteht bei einer mehrstufigen Biosynthese von Glycin aus Cholin als Zwischenprodukt durch Transaminierung von Betain mit Betain-Homocystein-Methylase. DMG und DMG-Na unterstützen nachweislich auch das Gewebe und die Zellenfunktionen, z. B. durch Verbesserung antioxidativer Kapazitäten und der Sauerstoffverwertung, Förderung der Geweberegeneration und Verbesserung der Immunantwort/Abwehr.

Erfindungsgemäß ist nicht nur der Einsatz von Dimethylglycin, sondern auch der Einsatz von dessen Salzen, Solvaten und Hydraten. Dabei handelt es sich bei den Salzen bevorzugt um pharmazeutisch annehmbare Salze von Dimethylglycin. Das Salz ist besonders bevorzugt ein wasserlösliches Salz mit einer Löslichkeit in Wasser von mindestens 10 g/l bei 20°C.

In einer bevorzugten Aufführungsform ist das Salz von Dimethylglycin ein Alkali-, Erdalkali- oder Ammoniumsalz von Dimethylglycin.

Beispiele sind Natrium-, Kalium-, Calcium-, Magnesium- und Ammoniumsalze. Bei den Ammoniumsalzen trägt das Ammoniumkation eine bis vier Alkylgruppen mit jeweils unabhängig voneinander 1 bis 4 Kohlenstoffatomen. Erfindungsgemäß bevorzugt sind das Natrium- und/oder Kaliumsalz von Dimethylglycin, insbesondere das Natriumsalz von Dimethylglycin, nämlich Natrium-N,N-dimethylglycinat (Natrium-dimethylglycinat, Na-DMG).

In einer alternativ bevorzugten Ausführungsform kann das Salz von Dimethylglycin das Salz einer anorganischen und/oder organischen Säure mit Dimethylglycin sein.

Beispiele für Salze von Dimethylglycin mit einer anorganischen Säure sind das Hydrochlorid, Hydrobromid, Hydroiodid, Hydrogensulfat, Sulfat, Hydrogensulfit, Sulfit, Hydrogencarbonat, Carbonat, Monophosphat, Diphosphat und Triphosphat von Dimethylglycin sowie Mischungen daraus. Insbesondere bevorzugt ist das Hydrochlorid von Dimethylglycin (Dimethylglycin-hydrochlorid, DMG-HCl).

Besonders bevorzugt ist Dimethylglycin und/oder ein Salz davon (B) ausgewählt aus Dimethylglycin, Natrium-dimethylglycinat, Dimethylglycin-hydrochlorid oder beliebigen Mischungen davon.

Bevorzugt enthält die erfindungsgemäße Zusammensetzung Dimethylglycin und/oder ein Salz von Dimethylglycin als chemischen Reinstoff, einschließlich der jeweiligen Solvate und Hydrate (z.B. des Dihydrats von Natriumdimethylglycinat), da so die Reinheit der Zusammensetzung erhöht und das Auftreten von unerwünschten Nebenwirkungen vermindert werden kann.

Bevorzugt enthält die erfindungsgemäße Zusammensetzung Dimethylglycin und/oder ein Salz von Dimethylglycin, insbesondere DMG, Na-DMG und/oder DMG-HCl in einer Menge von 0,0001 bis 35,0 Gew.-%, bevorzugt 0,001 bis 25,0 Gew.-%, stärker bevorzugt 0,01 bis 15,0 Gew.-%, noch stärker bevorzugt 0,1 bis 10,0 Gew.-%, und besonders bevorzugt 0,1 bis 5,0 Gew.-%.

Es ist ferner bevorzugt, dass die erfindungsgemäße Zusammensetzung Sildenafil und/oder ein Salz davon (A) sowie Dimethylglycin und/oder ein Salz davon (B) in einem molaren Verhältnis von 10000:1 bis 1:50, bevorzugt von 1000:1 bis 1:30, stärker bevorzugt von 100:1 bis 1:20, besonders bevorzugt von 10:1 bis 1:10 enthält. Das molare Verhältnis ist dabei das molare Verhältnis der Wirkstoffe, d.h. das molare Verhältnis von Sildenafil zu DMG.

Erfindungsgemäß wurde ein überraschender synergistischer Effekt für die Kombination von Sildenafil mit DMG zur Förderung der Vasodilatation und damit der Durchblutung gefunden. Die Erfinder konnten überraschend zeigen, dass DMG die Expression von endothelialer Stickstoffmonoxid-Synthase erhöht und die zelluläre Produktion von Stickstoffmonoxid (NO) induziert. Ohne an eine Theorie gebunden zu sein, lässt sich die synergistische Wirkung der Wirkstoffe durch eine simultane Erhöhung der eNOS-Expression durch DMG und eine Hemmung der PDE5-Aktivität durch Sildenafil erklären, wodurch die NO-abhängige Vasodilatation synergistisch gefördert wird.

Gemäß der vorliegenden Erfindung wurde überraschend gefunden, dass die Kombination von Sildenafil und/oder einem Salz von Sildenafil mit DMG und/oder einem Salz von DMG eine ausgezeichnete Wirksamkeit zur Förderung der Durchblutung zeigt und in hervorragender Weise zur Vorbeugung und/oder Behandlung von Haarausfall sowie zur Vorbeugung und/oder Behandlung von sexuellen Dysfunktionen geeignet ist.

Es hat sich dabei überraschenderweise gezeigt, dass DMG in der Kombination mit Sildenafil die Nebenwirkungen zu reduzieren scheint, die bei Behandlung mit Sildenafil als alleinigem Wirkstoff auftreten.

Die erfindungsgemäße Zusammensetzung kann zur topischen und/oder oralen Anwendung ausgestaltet sein.

Unter einer topischen Anwendung ist eine äußerliche Anwendung, insbesondere eine örtliche, äußerliche Anwendung zu verstehen.

Die Verabreichung kann lokale und/oder systemische Wirkung erzielen.

In bevorzugten Ausführungsformen ist die erfindungsgemäße Zusammensetzung zur topischen Anwendung ausgestaltet. Bevorzugt ist die erfindungsgemäße Zusammensetzung zur topischen kutanen Anwendung, d.h. zur topischen Anwendung auf der Haut ausgestaltet, besonders bevorzugt zur topischen Anwendung auf der Kopfhaut.

Die erfindungsgemäße Zusammensetzung kann in jeder zur oralen oder topischen Verabreichung geeigneten Darreichungsform vorliegen. Die Zusammensetzung kann entsprechend in einer flüssigen, festen oder halbfesten Darreichungsform vorliegen.

Die erfindungsgemäße Zusammensetzung zur oralen Anwendung liegt bevorzugt in flüssiger oder fester Form, insbesondere in fester Form vor.

Die erfindungsgemäße Zusammensetzung zur topischen Anwendung liegt bevorzugt in flüssiger oder halbfester Form vor.

Dementsprechend liegt die erfindungsgemäße Zusammensetzung in bevorzugten Ausführungsformen bei Raumtemperatur und insbesondere bei 20°C in flüssiger oder halbfester Form vor.

In anderen Ausführungsformen liegt die erfindungsgemäße Zusammensetzung in fester Form vor.

Erfindungsgemäß ist insbesondere für flüssige oder halbfeste Ausführungsformen bevorzugt, dass die Zusammensetzung eine Viskosität von 0,01 bis 10 Pa·s bei 20°C aufweist. Die Viskosität kann gemäß DIN 53019-1:2008-09 unter Verwendung eines Rheometers gemessen werden. Zum Beispiel kann die Viskosität unter Verwendung eines Rheometers des Typs MCR 301 (Anton Paar) bei 20°C mit einer Schergeschwindigkeit von 20 s⁻¹ unter Verwendung einer Kegel-Platte-Geometrie gemessen werden.

Bevorzugt weist die Zusammensetzung, insbesondere die flüssige oder halbfeste Zusammensetzung, einen pH-Wert im Bereich von 3,0 bis 6,0, bevorzugt 4,0 bis 5,5 auf. Der angegebene pH-Wert gilt bevorzugt bei Raumtemperatur, insbesondere bei 20°C, und kann mit einem pH-Meter gemessen werden, z.B. Mettler-Toledo SevenCompact S220. Falls die Zusammensetzung eine zu hohe Viskosität für die Messung des pH-Werts mit einem pH-Meter aufweist, kann der pH-Wert näherungsweise mit pH-Indikatorpapier bestimmt werden.

In dem bevorzugten pH-Bereich sind die erfindungsgemäßen Zusammensetzungen nicht nur chemisch, physikalisch und mikrobiologisch besonders stabil, sondern auch medizinisch und kosmetisch äußerst verträglich, insbesondere für eine topische Verabreichung. Besonders geeignete pH-Werte für die erfindungsgemäße Zusammensetzung sind: 4,0; 4,1; 4,2; 4,3; 4,4; 4,5; 4,6; 4,7; 4,8; 4,9; 5,0; 5,1; 5,2; 5,3; 5,4; und 5,4.

Der pH-Wert der Zusammensetzung wird bevorzugt eingestellt mithilfe eines oder mehrerer pH-Modifizierungsmittel. Geeignete pH-Modifizierungsmittel können Säuren, Basen und/oder Puffersysteme sein, um den pH-Wert der Zusammensetzung zu stabilisieren oder zu beeinflussen. Typische pH-Modifizierungsmittel gemäß der vorliegenden Erfindung sind Adipin-, Zitronen-, Äpfel-, Bernstein-, Wein-, Ascorbin-, Phosphor-, Milch- und Fumarsäure sowie die entsprechenden Salze, sowie Natriumalginat, Polyacrylsäure, Natriumcarbonat und Natriumbicarbonat. Im Zusammenhang mit pH-Modifizierungsmitteln bezieht sich der Begriff Salz auf Alkalimetallsalze oder Erdalkalimetallsalze, sofern nichts anderes angegeben ist.

Bevorzugt liegt Sildenafil und/oder ein Salz davon (A) vollständig gelöst in der erfindungsgemäßen flüssigen oder halbfesten Zusammensetzung vor. Vollständig gelöst bedeutet hierbei, dass die Komponente (A) in der Zusammensetzung bei 15-40°C molekular verteilt vorliegt und auch bei Lagerung über einen längeren Zeitraum, d.h. mehrere Tage bis Wochen, bei 15-40°C nicht ausfällt. Dies kann durch Bestimmung der Konzentration der gelösten Komponente (A), z.B. mittels HPLC, und/oder durch visuelle Untersuchung des makroskopischen oder mikroskopischen Erscheinungsbildes der Zusammensetzung überprüft werden. Insbesondere bei flüssigen klaren Zusammensetzungen kann ein Ausfallen einer Komponente durch visuelle Inspektion auf festes Präzipitat und/oder durch Evaluieren oder Messen der Trübung der Lösung bestimmt werden.

In bevorzugten Ausführungsformen umfasst die erfindungsgemäße Zusammensetzung, insbesondere die flüssige oder halbfeste Zusammensetzung, mindestens ein Lösungsmittel. Als Lösungsmittel kann ein für den Fachmann auf diesem Gebiet gängiges Lösungsmittel oder Lösungsmittelgemisch eingesetzt werden. Diese Lösungsmittel können vorzugsweise in einer Menge von 0,1 bis 99,0 Gew.% in der erfindungsgemäßen Zusammensetzung enthalten sein. Das Lösungsmittel ist bevorzugt ausgewählt aus Alkoholen, wässrigen Pufferlösungen, Ölen, Wasser oder Mischungen davon. In bevorzugten Ausführungsformen kann die erfindungsgemäße Zusammensetzung wasserbasiert sein. Das bedeutet, dass sie beispielsweise 45,0 bis 99,0 Gew.-% Wasser enthält.

Bei topischen Anwendungen kann je nach Wahl des Lösungsmittels die Penetration der Wirkstoffe verstärkt oder abgeschwächt werden und so eine verstärkte systemische Wirkung oder eine verstärkte topische Wirkung erreicht werden.

Bevorzugte Alkohole sind C₂-C₄-Alkohole mit 1-3 Hydroxygruppen und Mischungen davon, bevorzugt Ethanol, Isopropanol, 1-Propanol, Glycerin, Propylenglykol, 1,3-Propandiol, Butylenglykol oder Mischungen davon, insbesondere Ethanol und/oder Isopropanol.

Wässrige Pufferlösungen sind dem Fachmann bekannt und umfassen als Puffermittel mindestens eine schwache Säure und eine schwache Base, vorzugsweise ein konjugiertes Säure-Base-Paar. Der Pufferbereich der schwachen Säure und Base lässt sich aus dem pKₛ-Wert der Säure und optional der konjugierten Säure der schwachen Base berechnen und liegt bei einem konjugierten Säure-Base-Paar bei ca. pKₛ ± 1, insbesonder pKₛ ± 0,5. Vorzugweise überschneidet sich der Pufferbereich der wässrigen Pufferlösung mit einem pH-Bereich von 3,0-6,0, bevorzugt 4,0-5,5. Die Puffermittel, d.h. die mindestens eine schwache Säure und Base, liegen vorzugsweise in einer Gesamtkonzentration von 0,001 bis 2 M, vorzugsweise 0,01-1,0 M, insbesondere 0,1-0,8 M vor. Erfindungsgemäß bevorzugt sind Pufferlösungen ausgewählt aus Laktat-, Citrat-, Succinat-, Acetat- und Tartratpuffern und Mischungen davon, insbesondere Laktatpuffer.

In einer bevorzugten Ausführungsform umfasst die erfindungsgemäße Zusammensetzung mindestens 75 Gew.-% an wässrigem Puffer wie hierin definiert und 5-25 Gew.-% an Alkohol wie hierin definiert.

Erfindungsgemäß umfassen Öle pharmazeutisch annehmbaren Öle für die topische und/oder orale Anwendung, insbesondere pflanzliche Öle. Bevorzugte Öle umfassen Caprylic/Capric Triglyceride (Neutralöl), Kokusnussöl, Sojaöl, Sojalecithin, Sonnenblumenöl, Olivenöl, Palmöl, Rapsöl, Leinöl, Avocadoöl, Distelöl und Mischungen davon. Neben der Funktion als Lösungsmittel und/oder Träger haben Öle bei der topischen Anwendung auch eine rückfettende und pflegende Wirkung.

Die erfindungsgemäße Zusammensetzung, die in flüssiger oder halbfester Form vorliegt und insbesondere zur topischen Anwendung ausgestaltet ist, liegt bevorzugt als Gel, Creme, Salbe, Lotion, Lösung, Suspension oder Milch vor.

Gele bestehen aus gelierten Flüssigkeiten und werden mit geeigneten Quellmitteln wie beispielsweise Celluose-Derivaten, Stärken, Carbomeren, Gelatine, Xanthan, Bentonit, Agar, Tragant, Carrageen, Alginaten und/oder Pektin hergestellt.

Cremes sind halbfeste Zubereitungen, die in der Regel zur Anwendung auf der Haut oder Schleimhaut vorgesehen sind. Es handelt sich um mehrphasige Zubereitungen, die aus einer lipophilen und einer wässrigen Phase bestehen.

Salben sind halbfeste Zubereitungen zur äußerlichen Anwendung. Sie bestehen aus einer einphasigen Grundlage, in der feste oder flüssige Substanzen dispergiert sein können. Dies im Unterschied zu den Cremes, die mehrphasig sind und aus einer lipophilen und einer wässrigen Phase bestehen.

Lotionen sind Zubereitungen zur äußerlichen Anwendung auf der Haut mit einer flüssigen bis halbfesten Konsistenz.

Lösungen sind flüssige Zubereitungen, bei welchen die Wirk- und Hilfsstoffe in Wasser oder einem beliebigen anderen geeigneten Lösungsmittel (z.B. einem Öl) gelöst vorliegen.

Bevorzugt sind Lösungsmittel wie oben definiert. Lösungen können insbesondere bei der oralen Verabreichung kurz vor der Anwendung, beispielsweise aus einem Pulver, frisch hergestellt werden.

Suspensionen sind heterogene Stoffgemische, welche aus einer festen und flüssigen Phase bestehen. Die feste Phase kann sich mit der Zeit am Boden des Aufbewahrungsgefäßes absetzen. Deshalb sollten Suspensionen häufig direkt vor der Anwendung geschüttelt werden.

Wie oben erwähnt, ist eine weitere bevorzugte erfindungsgemäße Anwendung eine orale Verabreichung.

Bevorzugte orale Verabreichungsformen umfassen feste Darreichungsformen wie Tabletten, Kapseln, Dragees und Filmtabletten, Sirupe oder flüssige Formen wie Tropfen. Feste Darreichungsformen können mit modifizierter Wirkstofffreisetzung (z.B. verlängert oder verzögert) gefertigt werden.

Bevorzugt ist erfindungsgemäß eine orale Darreichungsform der Zusammensetzung als Tablette verpresst oder in einer Kapsel, z.B. einer Hartgelatinekapsel oder Weichgelatinekapsel.

Dabei kann die Zusammensetzung erfindungsgemäß ferner mindestens einen Trägerstoff und/oder mindestens einen Füllstoff umfassen.

Geeignete Trägerstoffe sind pharmazeutisch annehmbare Trägerstoffe und umfassen bevorzugt Pflanzenöle, wie beispielsweise Kokusnussöl, Sojaöl, Sojalecithin, Sonnenblumenöl, Olivenöl, Palmöl, Rapsöl und Leinöl; Bienenwachs; Zuckeralkohole, wie beispielsweise Sorbit, Mannit, Isomalt, Maltit, Lactit, Xylit und Erythrit; Monosaccharide wie Dextrose (Glucose), Mannose, Fructose; Disaccharide wie Maltose; und unverdauliche Kohlenhydrate wie beispielsweise Cellulose und Pektine.

Erfindungsgemäß bevorzugt werden ein oder mehrere Pflanzenöl(e) als Trägerstoff in Kapseln eingesetzt. Für Tabletten kommen vorzugsweise ein oder mehrere Zucker oder Zuckeralkohole, vorzugsweise Xylit zum Einsatz.

Geeignete Füllstoffe sind dem Fachmann bekannt. Sie werden insbesondere bei der Herstellung von oralen Verabreichungsformen wie Tabletten oder Kapseln mit geringen Wirkstoffmengen verwendet, um eine für die Herstellung nötige Größe oder Masse der Tablette zu gewährleisten. Bevorzugte Füllstoffe sind Stärke bzw. deren Bestandteile Amylopektin und Amylose und/oder Amylodextrin.

Stärke ist natürlicher Bestandteil von Kartoffeln, Maniok, Getreide (z.B. Weizen, Mais, Reis) sowie Hülsenfrüchten (z.B. Erbsen, Linsen, weiße oder rote Bohnen, Kichererbsen). Erfindungsgemäß ist Mais-, Kartoffel-, Weizen-, Reis- und/oder Hirsestärke insbesondere bevorzugt.

Amylopektin bezeichnet ein Polysaccharid aus α-D-Glucose-Monomeren, die stark verzweigte Strukturen bilden können. Die α-D-Glucose-Monomere sind dabei α-1,4-glycosidisch und an Verzweigungen α-1,6-glycosidisch miteinander verbunden.

Amylose bezeichnet ein Polysaccharid aus α-D-Glucose-Monomeren, die lineare Ketten mit helikaler Struktur bilden. Amylose hat eine molare Masse zwischen 100.000 und 1.000.000 g/mol, entsprechend etwa 1000 α-D-Glucose-Monomeren (bei Getreidestärken) bis 4500 α-D-Glucose-Monomeren (bei Kartoffelstärken), die α-1,4-glycosidisch miteinander verbunden sind. Bei hohen molaren Massen treten auch vereinzelte α-1,6-glycosidische Verzweigungen auf.

Amylodextrin ist ein lineares Dextrin und kann durch unvollständige Hydrolyse von Stärke durch verdünnte Säuren oder Amylase erhalten werden.

In einer bevorzugten Ausführungsform umfasst die erfindungsgemäße Zusammensetzung mindestens einen weiteren Wirkstoff (C). Der mindestens eine weitere Wirkstoff kann in Abhängigkeit von der zu behandelnden Indikation ausgewählt werden. In anderen Ausführungsformen ist der weitere Wirkstoff nicht in der erfindungsgemäßen Zusammensetzung enthalten, wird aber in entsprechender Kombination verabreicht. Wird die erfindungsgemäße Zusammensetzung in Kombination mit einer anderen Zusammensetzung umfassend einen weiteren Wirkstoff verabreicht, erfolgt die Verabreichung der beiden Zusammensetzung bevorzugt entsprechend den jeweiligen Dosierungsanweisungen der beiden Zusammensetzungen unabhängig voneinander. Eine zeitgleiche Verabreichung der beiden Zusammensetzungen ist selbstverständlich auch möglich.

Der mindestens eine weitere Wirkstoff (C) ist bevorzugt ausgewählt aus Menthol, Biotin, Zink PCA, Koffein, Niacinamid, Panthenol, Ectoin, Ubichinon-10, Taurin, Echinacea, Tocopherylacetat, NO-Donatoren, Amla-Extrakt, β-Carotin, Carnitin, Curcumin, Eisen, Folsäure, Granatapfel-Extrakt, Grüntee-Extrakt, Hirseextrakt, Hirseöl, Kürbiskernöl, Kreatin, L-Cystein, L-Lysin, Niacin, Pantolacton, Panthothensäure, Piperin, Procyanidin, Rosmarin-Extrakt, Sägepalme-Extrakt, Schachtelhalm-Extrakt, Selen, Silicium, Tomatenextrakt, Vitamin B1, Vitamin B2, Vitamin B6, Vitamin B12, Vitamin C, Vitamin E und beliebigen Kombinationen davon.

Der mindestens eine weitere Wirkstoff (C) ist bevorzugt ausgewählt aus Menthol, Biotin, Zink PCA, Koffein, Niacinamid, Panthenol, Ectoin, Ubichinon-10, Taurin, Echinacea, Tocopherylacetat und beliebigen Kombinationen davon.

Menthol ist ein monocyclischer Monoterpenalkohol und kann der erfindungsgemäßen Zusammensetzung als die Durchblutung stimulierender Wirkstoff zugesetzt werden. Zudem kann Menthol eine erfrischende sensorische Stimulierung der Haut bewirken.

Biotin, welches auch als Vitamin B7 oder Vitamin H bezeichnet wird, ist ein wasserlösliches Vitamin aus dem B-Komplex. Biotin kann erfindungsgemäß Haarausfall weiter vermindern und die Haut stärken.

Zink PCA ist das Zinksalz des L-Pyrrolidon-Carboxylats und kann als Substanz mit antimikrobieller Wirkung der erfindungsgemäßen Zusammensetzung zugesetzt werden.

Koffein ist ein zur Klasse der Methylxanthine gehörendes Methylxanthinalkaloid. Es ist eine bittere kristalline Substanz, kann als Purinderivat betrachtet werden und ist chemisch mit den Adenin- und Guaninbasen der Desoxyribonukleinsäuren und der Ribonukleinsäure verwandt. Der IUPAC-Name von Koffein ist 1,3,7-Trimethyl-3,7-dihydro-1H-purin-2,6-dion. Koffein ist bekannt für verschiedene pharmakologische Effekte. So ist es insbesondere bekannt für seine stimulierende Wirkung auf das zentrale Nervensystem. Es existiert eine wachsende Anzahl von Nachweisen für einen positiven Einfluss auf eine Reihe von unterschiedlichen Dysfunktionen.

Niacinamid (auch Nicotinamid) ist das Amid der Nicotinsäure und wird auch als Vitamin B3 bezeichnet. Niacinamid führt zu einer Verringerung von oxidativem Stress. Außerdem hat Niacinamid erfindungsgemäß eine das Haarwachstum stimulierende Wirkung.

Panthenol ist ein Provitamin, welches im Körper zu Pantothensäure (Vitamin B5) umgewandelt wird. Letzteres ist Teil des Coenzyms A und damit für den Hautstoffwechsel wichtig. Bei Einwirkung von Panthenol wird erfindungsgemäß die Hautelastizität und Feuchtigkeit weiter verbessert. Daneben werden Juckreiz und Entzündungen gelindert und die Wundheilung gefördert.

Ectoin ist eine zyklische Aminosäure und liegt in wässriger Lösung als mesomeriestabilisiertes Zwitterion vor. Es hat sich gezeigt, dass Ectoin vor UV-Strahlung schützt, die natürliche Struktur von Haaren stabilisiert und bei der Behandlung entzündlicher Krankheiten hilfreich sein kann.

Ubichinon-10 (Q10 oder Coenzym Q10) ist ein Chinon-Derivat. Das zum Ubichinon-Pool gehörende Q10 gilt als Antioxidans und wirkt erfindungsgemäß stabilisierend auf die Haut, Haare und Haarwurzel.

Taurin oder 2-Aminoethansulfonsäure wirkt erfindungsgemäß als Antioxidans ebenfalls weiter stabilisierend auf die Haut, Haare und Haarwurzel.

Echinacea wirkt erfindungsgemäß beruhigend auf die Haut und lindert auftretenden Juckreiz sowie Spannungen. Zudem kann Echinacea die Blutzirkulation der Haut anregen und so auch die Haarfollikel mit sauerstoff- und nährstoffreichem Blut versorgen, was sich weiter stabilisierend auf die Haare und insbesondere die Haarwurzel auswirkt.

Tocopherylacetat zeigt antioxidative Eigenschaften und wirkt erfindungsgemäß weiter stabilisierend auf die Haut, Haare und Haarwurzel.

NO-Donatoren wie L-Arginin und Nitroderivate (z.B. Nitroglyzerin) erhöhen die Stickstoffmonoxidproduktion, was zu einer Erweiterung der Blutgefäße und einer Verbesserung der Durchblutung führen kann. Somit ist bei korrekter Dosierung eine synergistische Wirkung mit Sildenafil und DMG zu erwarten.

Amla-Extrakt ist ein Extrakt aus der Indischen Stachelbeere (Phyllanthus emblica). β-Carotin ist eine Vorstufe von Vitamin A und wird deshalb auch als Provitamin A bezeichnet. Pantolacton entstammt der Gruppe der substituierten Lactone und regt erfindungsgemäß Wachstumsfaktoren von Haarwurzeln weiter an.

In einer bevorzugten Ausführungsform der Erfindung enthält die erfindungsgemäße Zusammensetzung mindestens einen weiteren Wirkstoff (C) wie oben definiert, bevorzugt ausgewählt aus Menthol, Biotin, Zink PCA, Niacinamid, Panthenol, Ectoin, Koffein, Ubichinon, Taurin, Echinacea, Tocopherylacetat und Kombinationen davon, jeweils in einem Anteil von 0,001 Gew.-% bis 10,0 Gew.-%, mehr bevorzugt 0,005 Gew.-% bis 7,50 Gew.-%, noch mehr bevorzugt von 0,01 Gew.-% bis 5,0 Gew.-%, insbesondere von 0,1 Gew.-% bis 3,0 Gew.-%, bezogen auf das Gesamtgewicht der Zusammensetzung.

Die erfindungsgemäße Zusammensetzung kann in bevorzugten Ausführungsformen wenigstens ein Additiv enthalten. Das wenigstens eine Additiv ist, insbesondere für die erfindungsgemäße Zusammensetzung zur topischen Anwendung, bevorzugt ausgewählt aus der Gruppe bestehend aus Rückfettern, Konservierungsmitteln, Stabilisatoren, Antioxidantien, Rheologiemodifikatoren, Verdickungsmitteln, Pflegemitteln, Farbstoffen und beliebigen Kombinationen hiervon. Für die erfindungsgemäße Zusammensetzung zur oralen Anwendung sind Additive besonders bevorzugt ausgewählt aus Konservierungsmitteln, Stabilisatoren, Antioxidantien, Farbstoffen und beliebigen Kombinationen hiervon.

Rückfetter, auch Rückfettungsmittel oder Überfettungsmittel genannt, sind lipophile Substanzen, die eine störende Auswirkung auf die epidermale Barrierefunktion verhindern können. Beispiele für Rückfetter sind Wollwachs, Squalen, flüssiges Paraffin, pflanzliche Öle, Silikone und Cetylpalmitat.

Konservierungsmittel sind Substanzen, die zur Konservierung verwendet werden, indem sie die Zusammensetzung zersetzende Mikroorganismen abtöten und/oder deren Wachstum hemmen. Vorzugsweise können die Konservierungsmittel ausgewählt sein aus der Gruppe bestehend aus Benzoesäure, Benzoesäurederivaten, Sorbinsäure, Sorbinsäurederivaten, Salicylsäure, Salicylsäurederivaten, Phenoxyethanol, Parabenen und Kombinationen hiervon. In einer bevorzugten Ausführungsform werden Natriumbenzoat und/oder Kaliumsorbat als Konservierungsmittel in der erfindungsgemäßen Zusammensetzung eingesetzt. Natriumbenzoat setzt in leicht saurem Milieu Benzoesäure und Kaliumsorbat Sorbinsäure frei. Beiden Säuren wird eine antimikrobielle Wirkung zugeschrieben.

Stabilisatoren können lichtempfindliche Komponenten gegenüber Strahlung schützen und sind bevorzugt UV-Absorber wie beispielsweise Benzophenonderivate.

Die Zugabe von Duftstoffen kann für einen angenehmen Geruch der Zusammensetzung sorgen, insbesondere für die erfindungsgemäße Zusammensetzung zur topischen Anwendung. Beispiele sind die dem Fachmann bekannten Parfums.

Ein Antioxidans oder Antioxidationsmittel ist eine chemische Verbindung, die eine Oxidation anderer Komponenten in der erfindungsgemäßen Zusammensetzung verlangsamt oder gänzlich verhindert. Als Antioxidantien kommen beispielsweise Zitronensäure, Ascorbinsäure und Butylhydroxyanisol in Frage.

Rheologiemodifikatoren und Verdickungsmittel können dabei helfen, die Applikationseigenschaften der erfindungsgemäßen Zusammensetzung beispielsweise für die topische Anwendung zu verbessern. Als Rheologiemodifikator und Verdickungsmittel kann die Zugabe von Kochsalz (Natriumchlorid) in Betracht gezogen werden. Durch die Zugabe von Kochsalz kann die Fließfähigkeit der erfindungsgemäßen Zusammensetzung in gewissen Grenzen beeinflusst und auf das nötige Maß eingestellt werden. Als Verdicker können zusätzlich Cellulose-derivate oder Polyacrylate eingesetzt werden.

Im Rahmen der Anmeldung sollen unter Pflegemitteln Substanzen verstanden werden, welche die Haut, insbesondere Kopfhaut pflegen. Hydrolysiertes Weizenprotein und Allantoin wirken pflegend auf Haut. Hydrolysiertes Weizenprotein hat hauptsächlich feuchtigkeitsspendende Eigenschaften.

Farbstoffe werden optional dazu verwendet, der erfindungsgemäßen Zusammensetzung eine charakteristische Farbe zu verleihen, so dass diese leicht von anderen Produkten unterschieden werden kann. Auch können Farbstoffe dazu beitragen, die Akzeptanz durch und/oder Compliance von Patienten zu verbessern.

Die erfindungsgemäße Zusammensetzung kann durch alle dem Fachmann bekannte Verfahren hergestellt werden.

Ein weiterer Aspekt der vorliegenden Erfindung bezieht sich auf die Verwendung der erfindungsgemäßen Zusammensetzung zur Vorbeugung und/oder Behandlung von Haarausfall und/oder von sexuellen Dysfunktionen.

Ein weiterer Aspekt der vorliegenden Erfindung bezieht sich auf die erfindungsgemäße Zusammensetzung zur Verwendung in einem Verfahren zur Vorbeugung und/oder Behandlung von Haarausfall und/oder von sexuellen Dysfunktionen.

Dabei ist der Haarausfall bevorzugt ausgewählt aus Alopecia areata (kreisrundem Haarausfall), Alopecia androgenetica (erblich bedingtem Haarausfall) bei Frauen und Männern, diffuser Alopezie (diffusem Haarausfall), altersbedingtem Haarausfall (senescent Alopecia), durch Chemotherapie bedingtem Haarausfall, Lichen planopilaris, Telogenem effluvium und frontaler fibrosierender Alopezie.

Besonders bevorzugt ist der zu behandelnde Haarausfall erblich bedingter Haarausfall (Alopecia androgenetica) und/oder altersbedingter Haarausfall (senescentAlopecia).

Für die Vorbeugung und/oder Behandlung von Haarausfall ist eine topische Anwendung der erfindungsgemäßen Zusammensetzung, insbesondere auf der Kopfhaut, besonders bevorzugt.

Neben der Vorbeugung und/oder Behandlung von Haarausfall verbessert die Verwendung der erfindungsgemäßen Zusammensetzung ferner den Zustand der Haare und/oder der Haut, und fördert den Stoffwechsel der Haare und/oder der Haut und das Haarwachstum.

Neben der Vorbeugung und/oder Behandlung von Haarausfall eignet sich die erfindungsgemäße Zusammensetzung insbesondere auch zur Vorbeugung und/oder Behandlung von sexuellen Dysfunktionen. Hierbei ist eine orale Anwendung und/oder eine topische Anwendung im Genitalbereich bevorzugt. Die sexuellen Dysfunktionen umfassen insbesondere männliche und weibliche sexuelle Erregungsstörungen. Bevorzugt ist die Verwendung in einem Verfahren zur Vorbeugung und/oder Behandlung von erektiler Dysfunktion und/oder weiblichen genitalen und/oder kombinierten Erregungsstörungen.

Es hat sich überraschenderweise gezeigt, dass DMG in der Kombination mit Sildenafil neben einer ausgezeichneten Wirksamkeit insbesondere die Nebenwirkungen zu reduzieren scheint, die bei Behandlung mit Sildenafil als alleinigem Wirkstoff auftreten. Dadurch wird die Sicherheit und Patientenzufriedenheit erhöht. Daneben ermöglicht das vorteilhafte Nebenwirkungsprofil eine wiederholte bzw. regelmäßige Behandlung, um beispielsweise eine langfristige Verbesserung des Zustands der Haare und/oder der Kopfhaut durch eine über einen längeren Zeitraum verbesserte Durchblutung zu erzielen.

Die Dosierung der erfindungsgemäß verwendeten Zusammensetzung ist von der zu behandelnden Indikation und dem zu behandelnden Patienten abhängig. Hier können u.a. Alter, Gewicht, Größe, Geschlecht, andere, insbesondere regelmäßig, eingenommene Medikamente, Schwangerschaft und/oder Vorerkrankungen eine Rolle spielen. Basierend auf derartigen Parametern kann die Dosierung vom Fachmann bestimmt werden. Die Zusammensetzungen können zur Behandlung und/oder Vorbeugung akuter und oder chronischer Zustände verabreicht werden. Insbesondere zur Behandlung chronischer Zustände kann eine Dauerbehandlung erfolgt.

Ein weiterer Aspekt der vorliegenden Erfindung betrifft einen Kit umfassend
(A) Sildenafil und/oder ein Salz davon, und
(B) Dimethylglycin und/oder ein Salz davon,
wobei (A) und (B) in getrennten Verabreichungsformen vorliegen.

Die Komponenten (A) und (B) sind dabei bevorzugt wie oben definiert. Die Zusammensetzung im Kit umfassend (A) und die Zusammensetzung im Kit umfassend (B) entsprechen dabei bevorzugt den oben genannten bevorzugten Ausführungsformen der erfindungsgemäßen Zusammensetzung, mit dem Unterschied, dass die Zusammensetzungen im Kit entweder (A) oder (B), jedoch nicht (A) und (B) enthalten.

Die Zusammensetzungen bzw. Formulierungen umfassend (A) bzw. (B) können zur topischen und/oder oralen Verabreichung vorgesehen sein, wobei die oben beschriebenen Verabreichungsformen bevorzugt sind. Beide Wirkstoffe können beispielsweise in Form einer Tablette verabreicht werden, oder beide Wirkstoffe können topisch verabreicht werden. Es ist aber auch möglich, einen Wirkstoff oral, z.B. als Tablette, und den entsprechend anderen Wirkstoff topisch, z.B. in Form einer Creme, Lotion, Suspension oder Shampoo-Grundlage zu verabreichen.

Die beiden Verabreichungsformen können gleichzeitig oder zeitlich versetzt voneinander verabreicht werden. Bei zeitlich versetzter Verabreichung beträgt der zeitliche Abstand vorzugsweise nicht mehr als eine Woche, bevorzugt nicht mehr als 3 Tage, stärker bevorzugt nicht mehr als 24 h und besonders bevorzugt 1 min-15 h. Die Reihenfolge der Verabreichung der beiden Wirkstoffe (A) und (B) ist dabei nicht besonders eingeschränkt. So ist es beispielsweise möglich, eine Tablette umfassend einen der Wirkstoffe am Morgen und eine Tablette umfassend den entsprechend anderen Wirkstoff am Abend einzunehmen. Genauso kann eine Lotion umfassend einem Wirkstoff am Morgen topisch angewendet werden und eine Tablette oder Lotion umfassend den entsprechend anderen Wirkstoff am Abend eingenommen (Tablette) bzw. topisch angewendet (Lotion) werden.

Erfindungsgemäß kann durch die Verwendung des Kits ein ähnlicher Effekt erzielt werden wie durch die Verwendung der erfindungsgemäßen Zusammensetzung umfassend beide Wirkstoffe.

### Beispiele

Anhand der nachfolgenden Zusammensetzungen soll die Erfindung verdeutlicht werden, ohne sie jedoch auf die speziellen Beispiele einschränken zu wollen.

### Beispiel 1: Formulierungen für erfindungsgemäße Lösungen

### Lösungsformulierung 1

1 Gew.-% Natrium-Dimethylglycin
1 Gew.-% Sildenafilcitrat
50 Gew.-% Ethanol
ad 100 Gew.-% gereinigtes Wasser

### Lösungsformulierung 2

0.0001 Gew.-% Natrium-Dimethylglycin
1.0 Gew.-% Sildenafilcitrat
50 Gew.-% Ethanol
ad 100 Gew.-% gereinigtes Wasser

### Lösungsformulierung 3

3.0 Gew.-% Natrium-Dimethylglycin
0.1 Gew.-% Sildenafilcitrat
5 Gew.-% Ethanol
ad 100 Gew.-% gereinigtes Wasser

Zur Herstellung der jeweiligen Formulierung werden Ethanol und gereinigtes Wasser gemischt, anschließend werden Natrium-Dimethylglycin und Sildenafilcitrat nacheinander dazugegeben und in der Mischung gelöst.

Die Zusammensetzungen können topisch auf die Haut aufgetragen oder peroral eingenommen werden. Eine topische Anwendung ist bevorzugt. Lösungsformulierung 1 stellt eine erfindungsgemäß besonders bevorzugte Formulierung dar.

### Beispiel 2: Formulierungen für erfindungsgemäße Tabletten

### Tablettenformulierung 1

20 Gew.-% Sildenafilcitrat
0.1 Gew.-% Natrium-Dimethylglycin
1% Gew.-% Magnesiumstearat
1% Gew.-% Siliziumdioxid
ad 100 Gew.-% mikrokristalline Cellullose

### Tablettenformulierung 2

0.1 Gew.-% Sildenafilcitrat
35 Gew.-% Natrium-Dimethylglycin
1 Gew.-% Magnesiumstearat
1 Gew.-% Siliziumdioxid
ad 100 Gew.-% mikrokristalline Cellulose

Die Komponenten werden gemischt und zu Tabletten gepresst. Die Einnahme erfolgt oral. Tablettenformulierung 1 stellt erfindungsgemäß eine bevorzugte Formulierung dar.

Folgende Items fassen die Erfindung zusammen:
1. Zusammensetzung umfassend
   (A) Sildenafil und/oder ein Salz davon, und
   (B) Dimethylglycin und/oder ein Salz davon.
2. Zusammensetzung nach Anspruch 1, wobei Sildenafil und/oder ein Salz davon (A) ein Salz von Sildenafil ist, bevorzugt ein pharmazeutisch annehmbares Salz von Sildenafil, insbesondere Sildenafilcitrat.
3. Zusammensetzung nach einem der vorhergehenden Items, wobei die Zusammensetzung 0,001 bis 20,0 Gew.-%, bevorzugt 0,001 bis 15,0 Gew.-%, stärker bevorzugt 0,01 bis 10,0 Gew.-%, noch stärker bevorzugt 0,1 bis 5,0 Gew.-% und besonders bevorzugt 0,1 bis 1,5 Gew.-% Sildenafil und/oder ein Salz davon (A) enthält.
4. Zusammensetzung nach einem der vorhergehenden Items, wobei das Salz von Dimethylglycin (B) ein Alkali-, Erdalkali- oder Ammoniumsalz von Dimethylglycin oder das Salz einer anorganischen und/oder organischen Säure mit Dimethylglycin ist.
5. Zusammensetzung nach einem der vorhergehenden Items, wobei Dimethylglycin und/oder ein Salz davon (B) ausgewählt ist aus Dimethylglycin, Natrium-dimethylglycinat und Dimethylglycin-hydrochlorid.
6. Zusammensetzung nach einem der vorhergehenden Items, wobei die Zusammensetzung 0,0001 bis 35,0 Gewichtsprozent (Gew.-%), bevorzugt 0,001 bis 25,0 Gew.-%, stärker bevorzugt 0,01 bis 15,0 Gew.-%, noch stärker bevorzugt 0,1 bis 10,0 Gew.-%, und besonders bevorzugt 0,1 bis 5,0 Gew.-% Dimethylglycin und/oder ein Salz von Dimethylglycin enthält.
7. Zusammensetzung nach einem der vorhergehenden Items, wobei die Zusammensetzung Sildenafil und/oder ein Salz davon (A) sowie Dimethylglycin und/oder ein Salz davon (B) in einem molaren Verhältnis von 10000:1 bis 1:50, bevorzugt von 1000:1 bis 1:30, stärker bevorzugt von 100:1 bis 1:20, besonders bevorzugt von 10:1 bis 1:10 enthält.
8. Zusammensetzung nach einem der vorhergehenden Items, wobei die Zusammensetzung bei 20°C in flüssiger oder halbfester Form vorliegt.
9. Zusammensetzung nach einem der vorhergehenden Items, wobei die Zusammensetzung eine Viskosität von 0,01 bis 10 Pa·s bei 20°C aufweist.
10. Zusammensetzung nach einem der vorhergehenden Items, wobei die Zusammensetzung einen pH-Wert im Bereich von 3,0 bis 6,0, bevorzugt 4,0 bis 5,5 aufweist.
11. Zusammensetzung nach einem der vorhergehenden Items, wobei Sildenafil und/oder ein Salz davon (A) vollständig gelöst in der Zusammensetzung vorliegt.
12. Zusammensetzung nach einem der vorhergehenden Items, wobei die Zusammensetzung mindestens ein Lösungsmittel enthält, wobei das Lösungsmittel bevorzugt ausgewählt ist aus Alkoholen, wässrigen Pufferlösungen, Ölen, Wasser oder Mischungen davon.
13. Zusammensetzung nach Item 12, wobei die Zusammensetzung mindestens 75 Gew.- % an wässrigem Puffer und 5-25 Gew.-% an Alkohol enthält.
14. Zusammensetzung nach einem der vorhergehenden Items, wobei die Zusammensetzung ein Gel, Creme, Salbe, Lotion, Lösung, Suspension oder Milch ist.
15. Zusammensetzung nach einem der vorhergehenden Items, wobei die Zusammensetzung zur topischen Anwendung ausgestaltet ist.
16. Zusammensetzung nach Item 15, wobei die Zusammensetzung zur topischen Anwendung auf der Haut ausgestaltet ist, insbesondere zur topischen Anwendung auf der Kopfhaut.
17. Zusammensetzung nach einem der Items 1-13, wobei die Zusammensetzung zur oralen Anwendung ausgestaltet ist.
18. Zusammensetzung nach Item 17, wobei die Zusammensetzung ein Gel, eine Lösung, eine Suspension, eine Milch oder ein Sirup ist.
19. Zusammensetzung nach Item 17, wobei die Zusammensetzung in fester Form vorliegt.
20. Zusammensetzung nach Item 19, wobei die Zusammensetzung in Form von Tabletten, Kapseln, Dragees oder Filmtabletten, bevorzugt in Form von Tabletten oder Kapseln, vorliegt.
21. Zusammensetzung nach einem der vorhergehenden Items, wobei die Zusammensetzung ferner mindestens einen weiteren Wirkstoff (C) umfasst.
22. Zusammensetzung nach Item 21, wobei der Wirkstoff (C) ausgewählt ist aus Menthol, Biotin, Zink PCA, Koffein, Niacinamid, Panthenol, Ectoin, Ubichinon-10, Taurin, Echinacea, Tocopherylacetat und Kombinationen davon.
23. Zusammensetzung nach einem der vorhergehenden Items, wobei die Zusammensetzung wenigstens ein Additiv enthält, ausgewählt aus Rückfettern, Konservierungsmitteln, Stabilisatoren, Antioxidantien, Rheologiemodifikatoren, Verdickungsmitteln, Pflegemitteln, Farbstoffen und Kombinationen hiervon.
24. Zusammensetzung nach einem der vorhergehenden Items zur Verwendung in einem Verfahren zur Vorbeugung und/oder Behandlung von Haarausfall und/oder von sexuellen Dysfunktionen.
25. Zusammensetzung nach Item 24 zur Verwendung in einem Verfahren zur Vorbeugung und/oder Behandlung von Haarausfall, wobei der Haarausfall bevorzugt ausgewählt ist aus Alopecia areata (kreisrundem Haarausfall), Alopecia androgenetica (erblich bedingtem Haarausfall) bei Frauen und Männern, diffuser Alopezie (diffusem Haarausfall), altersbedingtem Haarausfall (senescentAlopecia), durch Chemotherapie bedingtem Haarausfall, Lichen planopilaris, Telogenem effluvium und frontaler fibrosierender Alopezie, insbesondere aus Alopecia androgenetica und senescent Alopecia.
26. Zusammensetzung nach Item 25, wobei die Zusammensetzung zur topischen Anwendung, insbesondere zur topischen Anwendung auf der Kopfhaut ausgestaltet ist.
27. Zusammensetzung nach Item 24 zur Verwendung in einem Verfahren zur Vorbeugung und/oder Behandlung von sexuellen Dysfunktionen, wobei die sexuellen Dysfunktionen bevorzugt ausgewählt sind aus männlichen und weiblichen sexuellen Erregungsstörungen, insbesondere aus erektiler Dysfunktion und/oder weiblichen genitalen und/oder kombinierten Erregungsstörungen.
28. Kit umfassend
   (A) Sildenafil und/oder ein Salz davon, und
   (B) Dimethylglycin und/oder ein Salz davon,
   wobei (A) und (B) in getrennten Verabreichungsformen vorliegen.
29. Kit nach Item 28 zur Verwendung in einem Verfahren zur Vorbeugung und/oder Behandlung von Haarausfall und/oder von sexuellen Dysfunktionen.

## Patentansprüche

1. Zusammensetzung umfassend
(A) Sildenafil und/oder ein Salz davon, und
(B) Dimethylglycin und/oder ein Salz davon.

2. Zusammensetzung nach Anspruch 1, wobei Sildenafil und/oder ein Salz davon (A) ein Salz von Sildenafil ist, bevorzugt ein pharmazeutisch annehmbares Salz von Sildenafil, insbesondere Sildenafilcitrat.

3. Zusammensetzung nach einem der vorhergehenden Ansprüche, wobei die Zusammensetzung 0,001 bis 20,0 Gew.-%, bevorzugt 0,001 bis 15,0 Gew.-%, stärker bevorzugt 0,01 bis 10,0 Gew.-%, noch stärker bevorzugt 0,1 bis 5,0 Gew.-% und besonders bevorzugt 0,1 bis 1,5 Gew.-% Sildenafil und/oder ein Salz davon (A) enthält.

4. Zusammensetzung nach einem der vorhergehenden Ansprüche, wobei das Salz von Dimethylglycin (B) ein Alkali-, Erdalkali- oder Ammoniumsalz von Dimethylglycin oder das Salz einer anorganischen und/oder organischen Säure mit Dimethylglycin ist, wobei Dimethylglycin und/oder ein Salz davon (B) bevorzugt ausgewählt ist aus Dimethylglycin, Natrium-dimethylglycinat und Dimethylglycin-hydrochlorid.

5. Zusammensetzung nach einem der vorhergehenden Ansprüche, wobei die Zusammensetzung 0,0001 bis 35,0 Gewichtsprozent (Gew.-%), bevorzugt 0,001 bis 25,0 Gew.-%, stärker bevorzugt 0,01 bis 15,0 Gew.-%, noch stärker bevorzugt 0,1 bis 10,0 Gew.-%, und besonders bevorzugt 0,1 bis 5,0 Gew.-% Dimethylglycin und/oder ein Salz von Dimethylglycin enthält.

6. Zusammensetzung nach einem der vorhergehenden Ansprüche, wobei die Zusammensetzung bei 20°C in flüssiger oder halbfester Form vorliegt,
wobei die Zusammensetzung bevorzugt eine Viskosität von 0,01 bis 10 Pa·s bei 20°C aufweist.

7. Zusammensetzung nach einem der vorhergehenden Ansprüche, wobei die Zusammensetzung einen pH-Wert im Bereich von 3,0 bis 6,0, bevorzugt 4,0 bis 5,5 aufweist.

8. Zusammensetzung nach einem der vorhergehenden Ansprüche, wobei Sildenafil und/oder ein Salz davon (A) vollständig gelöst in der Zusammensetzung vorliegt, und/oder
wobei die Zusammensetzung mindestens ein Lösungsmittel enthält, wobei das Lösungsmittel bevorzugt ausgewählt ist aus Alkoholen, wässrigen Pufferlösungen, Ölen, Wasser oder Mischungen davon.

9. Zusammensetzung nach einem der vorhergehenden Ansprüche, wobei die Zusammensetzung ein Gel, Creme, Salbe, Lotion, Lösung, Suspension oder Milch ist.

10. Zusammensetzung nach einem der vorhergehenden Ansprüche, wobei die Zusammensetzung zur topischen Anwendung ausgestaltet ist, bevorzugt zur topischen Anwendung auf der Haut, insbesondere zur topischen Anwendung auf der Kopfhaut.

11. Zusammensetzung nach einem der Ansprüche 1-8, wobei die Zusammensetzung zur oralen Anwendung ausgestaltet ist.

12. Zusammensetzung nach Anspruch 11, wobei die Zusammensetzung ein Gel, eine Lösung, eine Suspension, eine Milch oder ein Sirup ist, oder
wobei die Zusammensetzung in fester Form vorliegt, bevorzugt in Form von Tabletten, Kapseln, Dragees oder Filmtabletten, besonders bevorzugt in Form von Tabletten oder Kapseln.

13. Zusammensetzung nach einem der vorhergehenden Ansprüche zur Verwendung in einem Verfahren zur Vorbeugung und/oder Behandlung von Haarausfall und/oder von sexuellen Dysfunktionen.

14. Kit umfassend
(A) Sildenafil und/oder ein Salz davon, und
(B) Dimethylglycin und/oder ein Salz davon,
wobei (A) und (B) in getrennten Verabreichungsformen vorliegen.
